# EUROPEAN PATENT SPECIFICATION

(11) **EP 2 800 741 B1**
(45) Date of publication and mention of the grant of the patent: **15.03.2017**
(21) Application number: 13709997.4
(22) Date of filing: 04.01.2013
(51) Int. Cl.: C07D 235/18, A61K 31/4184, A61Q 17/00

(54) **DUALISTIC MOLECULES HAVING UV RADIATION FILTRATING ABILITY AT WIDE SPECTRUM AND POTENT DAMPING ACTIVITY OF THE REACTIVITY OF FREE RADICALS (RADICALS SCAVENGING)**
NEUE DUALISTISCHE MOLEKÜLE MIT UV-STRAHLUNGSFILTERUNGSFÄHIGKEIT IN EINEM BREITEN SPEKTRUM UND WIRKUNGSVOLLER DÄMPFUNGSAKTIVITÄT DER REAKTIVITÄT FREIER RADIKALE (RADIKALSPÜLUNG)
NOUVELLES MOLÉCULES À DOUBLE FONCTION AYANT UNE CAPACITÉ DE FILTRATION DU RAYONNEMENT UV SUR UN LARGE SPECTRE ET UNE PUISSANTE ACTIVITÉ D'ATTÉNUATION DE LA RÉACTIVITÉ DE RADICAUX LIBRES (PIÉGEAGE DE RADICAUX)

(30) Priority: 05.01.2012 IT VR20120003
(43) Date of publication of application: 12.11.2014
(73) Proprietor: Università Degli Studi Di Ferrara, 44121 Ferrara (IT)
(72) Inventor: MANFREDINI, Stefano, I-44121 Ferrara (IT); VERTUANI, Silvia, I-44121 Ferrara (IT); SCALAMBRA, Emanuela, I-44121 Ferrara (IT)
(74) Representative: Feltrinelli, Secondo Andrea
(86) International application number: PCT/IB2013/000013
(87) International publication number: WO 2013/102843

(56) References cited:
- EP-A1- 0 884 046
- EP-A1- 1 167 358
- WO-A2-2012/108689
- DE-A1- 4 302 796
- US-A- 2 995 540
- US-A1- 2005 136 014
- AYDIN S ET AL: "ANALGESIC AND ANTISPASMODIC ACTIVITIES OF 2-(2-NITRO-PHENYL)-1H-BENZIMIDAZOLE 5-CARBOXYLIC ACID: EVIDENCE FOR THE IMPORTANCE OF THE 2-(O-SUBSTITUTED PHENYL) GROUP", DIE PHARMAZIE, GOVI VERLAG PHARMAZEUTISCHER VERLAG GMBH, ESCHBORN, DE, vol. 58, no. 6, 1 January 2003 (2003-01-01), pages 405-408, XP001207174, ISSN: 0031-7144
- HAKAN G ET AL: "Synthesis of some new benzimidazole-5(6)-carboxylic acids", JOURNAL HETEROCYCLIC CHEMISTRY, vol. 32, 1995, pages 1767-1773, XP002695652,

## Description

### TECHNICAL FIELD OF THE INVENTION

The present invention refers to a compound with structure 2-phenylbenzimidazole of formula I and/or a pharmaceutically acceptable salt thereof, wherein n is 1, 2 or 3 and in which R is H, a carboxyl radical (-COOH) or a sulphonic radical (-SO₃H), used in pharmaceutical and/or cosmetic formulations and/or in medical devices used as UV filters and agents counteracting the free radicals.

### STATE OF THE ART

Skin colour is the result of an evolutionary adaptation to exposure to ultraviolet rays. Skin, therefore, is very dark or very light, depending on the intensity of the UV rays, to allow appropriate photo-protection against radiation itself.

The concept of beauty has continued to change over the centuries from a Renaissance model, where light skin was synonymous with beauty, wealth and health, to a current model, where tanned skin alludes to the possibility to stay in the open air and, therefore, contrarily, has become synonymous with health, beauty and healthy living.

This change in customs, in addition to the advantages offered in the correction of small skin blemishes, has led to a strong propensity to exposure to the sun, which, however, sometimes is done in excess and often without adequate photo-protection.

The increase in photo-exposure, alongside a scarcity of effective molecules in the protection against the negative effects of the sun's rays, has led to the current interest in the problems associated with damage from UV radiation.

Incorrect exposure to the sun's rays and to radiation causing a suntan in general, indeed, can become dangerous since it involves damage both immediately and in the long term.

Indeed, it is thought that a very high percentage of shrinkage and pathological phenomena that the skin suffers over time, considered in the past to be the effect of chronological ageing, are, on the other hand, attributable to photoaging.

In order to avoid this problem, a correct use of sun filters could not only prevent burns and erythemas, but could also prevent the chronic effects deriving from the exposure to UV rays such as photocarcinogenesis, photoaging and immunosuppression.

The skin is the part of the body most exposed to external oxidising agents that can promote the formation of free radicals, in particular ultraviolet rays UV.

The ultraviolet radiation that reaches our skin can be distinguished into UVB (from 290 nm to 320 nm) and UVA (from 320 nm to 400 nm). The former are responsible for direct damage, like sun erythema, whereas the latter cause indirect damage, due, in most cases, to the formation of oxidising species.

Sun filters should, therefore, have a wide spectrum to ensure skin protection both against UVB and UVA rays.

In particular, the effects of UVA radiation are not perceived by the person right away and could go unnoticed, over time causing serious alterations. Among the main causes of damage from UVA there is the formation of free radicals of oxygen (ROS), highly reactive molecules that attack the polyunsaturated chains of fatty acids triggering a chain reaction that causes the oxidation of the lipids. This translates into damage particularly to cellular membranes that become more permeable and lose their efficiency. Consequently, the oxidising stress causes early ageing of cells and tissues, compromising their functionality.

Early ageing of skin, with the appearance of wrinkles, dark stains and other undesired blemishes, is one of the first signs of the presence of a situation of oxidising stress. The damage to the skin caused by ROS, however, can also extend to the loss of the barrier function of the stratum corneum, to the promotion of inflammatory processes, to erythema, up to cancer.

The skin, indeed, possesses a physiological defence mechanism from oxidising stress caused by exposure to the sun's rays. However, when sun exposure is excessive, the body is no longer able to neutralise the radical species generated by the UV rays, triggering the photoaging, immunosuppression and photocarcinogenesis mechanisms indicated above.

The UV filters used in pharmaceutical and/or cosmetic products form, in general, a protective layer on the surface of the skin and absorb the solar radiation themselves, thus preventing it from penetrating into the deeper layers of the skin.

Once the filtering molecule has absorbed the energy of solar radiation and in this way it has passed to an excited level, one of the methods for dispersing this energy is a transformation or a degradation of the molecule itself, which thus becomes photo-unstable (Serge Forestier, J. Am. Acad. Dermatol. "Rationale for sunscreen development" 2008; 58:S133-8). This modification can trigger the formation of oxidising radical species. Therefore, in addition to the loss of filtering power, there is possible cell damage deriving from the formation of such reactive species of oxygen (Nick Serpone, Angela Salinaro, Alexei V.Emeline, Satoshi Horikoshi, Hisao Hidaka and Jincai Zhao, "An in vitro systematic spectroscopic examination of the photostabilities of a random set of commercial sunscreen lotions and their chemical UVB/UVA active agents", Photochem. Photobiol. Sci. 2002, 1, 970-981).

As indicated earlier, it is mainly UVA radiation that generates the reactive species of oxygen that damage the cell structures of all of the layers of the skin, attacking the vital biomolecules of the cells themselves.

The formulation of cosmetic and/or pharmaceutical products can have a profound influence on the filtering capability of a molecule, in particular the pH and the solvents used directly influence the sun protection factor (SPF), the maximum wavelength absorbable (λ max) and the stability of the product.

The SPF value is a protection indicator that is well-known to measure the burn protection capability of a formulation. Since UVB rays are the main cause of burns and of the formation of erythema, SPF is consequently an indicator of the filtering effectiveness against UVB rays.

For many years the Applicant has been involved in the development of new molecules, so-called dualistic" capable of counteracting the sun damage generated by UV rays with a dual mechanism: on the one hand filtering radiation and on the other hand damping the reactivity of (scavenging) the free radicals produced by the radiation itself. Indeed, following a general screening carried out on commercial solar filters, in order to evaluate their filtering and/or anti-oxidant capability, it has been found that none of the molecules currently on the market simultaneously possesses such filtering and anti-oxidant capabilities at a significant level.

### PURPOSES OF THE INVENTION

A purpose of the present invention is to improve the state of the art.

Another purpose of the present invention is to provide a compound comprising dualistic molecules provided with the greatest possible filtering capability of UV radiation and with anti-oxidant activity.

A further purpose of the present invention is to provide a compound comprising dualistic molecules that can easily be used in the field of pharmaceuticals and/or cosmetics.

In accordance with an aspect of the present invention a compound with structure 2-phenylbenzimidazole of formula I and/or a pharmaceutically acceptable salt thereof is foreseen, according to claim 1.

In accordance with a further aspect of the present invention a pharmaceutical or cosmetic formulation or a medical device comprising a compound with structure 2-phenylbenzimidazole of formula I and/or a pharmaceutically acceptable salt thereof are foreseen, according to claim 3. The dependent claims refer to preferred and advantageous embodiments of the invention.

### EMBODIMENTS OF THE INVENTION

The present invention refers to a compound that acts as a water-soluble UV filter.

In the panorama of solar filters currently on the market there are not many water-soluble molecules. Usually, indeed, to give adequate solar protection and good compliance it is advantageous to have a mixture of water-soluble and liposoluble filters.

Among the few water-soluble UV filters available on the market, one of these is 2-phenyl-1H-benzimidazole-5-sulfonic acid and its pharmaceutically acceptable salts. This is a filter that is effective against UVB rays, which however does not offer UVA protection and completely lacks anti-oxidant activity.

By suitably modifying the structure of 2-phenyl-1H-benzimidazole-5-sulfonic acid, by introducing different functional groups into different positions of the phenyl substituent and modifying the fraction of sulfonic acid, it has surprisingly emerged that not only is the molecule obtained provided with anti-oxidant activity, but the protective spectrum against UV radiation extended from UVB to UVA and UVB, providing a wide-spectrum molecule never previously described, as better specified hereafter.

Such a result is totally unexpected since it was proposed with this modification to implement anti-oxidant activity on a known UVB filter. Whilst it is known that substituents introduced on the aromatic nucleus of substances with UV ray filtering activity can change the wavelength at which absorption takes place, it is not obvious that this takes place with widening of the spectrum. For example, the introduction of hydroxyl groups into the structure of bis-ethylhexyloxyphenol methoxyphenyl triazine, Tinosorb-S, a filter that absorbs in the UVA/UVB field, moves the filtering activity into UVA (Clinical Guide to sunscreens and photoprotection, Lin and Draelos eds, 2009, N.Y.). Therefore, when a substitution is carried out, it cannot be predicted whether such a substitution will keep the wavelength and extent of absorption in the UVB field, just like in UVA.

In particular, the present invention concerns a compound with structure 2-phenylbenzimidazole containing at least one hydroxyl group on the phenyl portion, or a pharmaceutically acceptable salt thereof, of formula I: 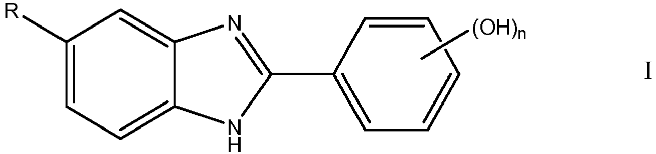 in which n is 1, 2 or 3 and R can be H (according to a comparative example thereof), a carboxyl radical (-COOH) or sulfonic radical (-SO₃H).

When R is -COOH or -SO₃H, the present invention also comprises the salts of these acids.

Such salts are preferably the salts of alkaline metals, in particular sodium or potassium salts, or ammonium salts, in particular the salts of triethanolamine, of the corresponding carboxyl and sulfonic acids.

Various synthetic processes for preparing aryl-benzimidazoles, such as the compound of formula I, are known. The compounds of formula I according to the invention, therefore, can be obtained by any process suitable for this purpose.

A process that has proven particularly effective and economic is the one described in J. Med. Chem. 2002, 45, pages 3576-3578, in which a 1,2-phenylenediamine or a derivative thereof of formula II: 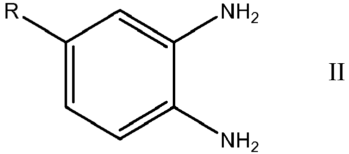 in which R is H, a carboxyl radical (-COOH) or a sulphonic radical (-SO₃H), is made to react in the presence of sodium hydrogen sulfite (NaHSO₃) with a substituted benzaldehyde of formula III: 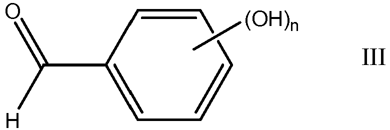 in which n is 1, 2 or 3.

The reaction was carried out using an alcohol as solvent, preferably methanol or ethanol.

The 1,2-phenylenediamine (II) and the substituted benzaldehyde (III) were used 1:1 ratio. The reaction was carried out in the presence of sodium hydrogen sulfite in variable ratio between 1:1 and 2:1 with respect to the 1,2-phenylenediamine (II), preferably in 2:1 ratio.

The reaction mixture was heated to a temperature between 60°C and 90°C, preferably between 75°C and 80°C; such a temperature was maintained for 12 - 24 hours.

The raw product was purified through filtration and subsequent washing with a suitable organic solvent and/or acid solution and/or through liquid chromatography.

A method for synthesising the compound of formula I according to the present invention is disclosed. Such a method is particularly advantageous for "bulk" production of the compound in question.

Such a method comprises a step of providing a reaction mixture comprising a phenylenediamine or a derivative thereof of formula II, ethanol, NaHSO₃ and a substituted benzaldehyde of formula III, or providing a reaction mixture comprising a phenylenediamine or a derivative thereof of formula II, water, NaHSO₃ and a substituted benzaldehyde of formula III, a step of agitating the reaction mixture, a step of heating the reaction mixture, and a step of filtering the mixture to obtain the compound of formula I.

Such a method foresees the mixing, during the provision step, 1-20 g of phenylenediamine or a derivative thereof of formula II, 10-200 ml of ethanol, 15-50 ml of NaHSO₃ and 1-20 g of substituted benzaldehyde of formula III or 10-40 g of phenylenediamine or a derivative thereof of formula II, 500-1000 ml of water, 60-90 ml of NaHSO₃ and 10-30 g of substituted benzaldehyde of formula III.

Such a method also comprises a step of diluting the reaction mixture comprising ethanol with water obtaining a diluted reaction mixture. Such a diluting step takes place before the heating step; the agitating step and the heating step can be simultaneous.

Such a method thereafter comprises a step of cooling the reaction mixture which can be followed by a step of leaving the reaction mixture itself to rest.

The method also comprises a step of washing the reaction mixture or a filtered solid component thereof with hydrochloric acid.

Further washing with water and/or acetone can follow in order to obtain the compound of formula I.

In a version, such a methodology foresees that a mixture of ethanol and water be used as solvent, heating the reaction mixture to a temperature comprised between 70°C and 90°C or to 78°C at a later time with respect to the conventional methodology given above.

Such a method, in an embodiment thereof, comprises a step of providing a reaction mixture comprising for example 3,4-diaminobenzoic acid, ethanol, NaHSO₃ 40% and for example dihydroxybenzaldehyde.

In particular, such a step comprises mixing 1-20 g of 3,4-diaminobenzoic acid, 10-200 ml of ethanol, 15-50 ml of NaHSO₃ and 1-20 g of dihydroxybenzaldehyde.

In a particular example, such a step comprises introducing into a 1000 ml balloon 10 g of 3,4-diaminobenzoic acid, 100 ml of ethanol, 34 ml of NaHSO₃ 40% and 9,08 g of dihydroxybenzaldehyde.

According to such an example, ethanol is present at a concentration equal to 10 ml on a gram of substituted phenylenediamine of formula II or of 3,4-diaminobenzoic acid; water is present at a concentration equal to 40 ml on a gram of substituted phenylenediamine of formula II or 3,4-diaminobenzoic acid; NaHSO₃ 40% is present at a concentration equal to 3,4 ml on a gram of substituted phenylenediamine of formula II or 3,4-diaminobenzoic acid. Such a method also comprises a step of agitating the reaction mixture; in particular such an agitating step takes place at room temperature for 2 hours. The agitation time can be comprised between 30 minutes and 5 hours.

The resulting mixture is very pasty: the method thus comprises a step of diluting the reaction mixture with water. In particular, in such a diluting step 200-800 ml or, in particular, 400 ml of water are added.

Such a diluting step also comprises a step of agitating and/or a step of heating the diluted reaction mixture to a temperature above 70°C or 78°C or comprised between 75°C and 78°C.

Such agitating and/or heating steps take place for two hours or for a period comprised between 1 hour and 18 hours.

The method also comprises a step of cooling the mixture and/or a step of leaving the mixture to rest; such a step of leaving to rest can take place overnight.

The method also comprises a step of filtering the mixture. Moreover, the filtered mixture is washed with hydrochloric acid.

Such a washing step comprises adding 300 ml of HCl 1N.

The method then comprises a further step of filtering the washed mixture.

In a further version, there is a method for synthesising the compound of formula I in which, with respect to the conventional method given above, the reaction solvent is substituted with water.

The heating step takes place immediately; the heating step takes place at a temperature comprised between 70°C and 90°C or at 78°C.

Such a method comprises a step of providing a phenylenediamine or a derivative thereof of formula II, water, NaHSO₃ and a substituted benzaldehyde of formula III. Such a method, in an embodiment thereof, comprises a step of providing a reaction mixture comprising for example 3,4-diaminobenzoic acid, water, NaHSO₃ 40% and for example dihydroxybenzaldehyde.

In particular, such a provision step comprises for example mixing 10-40 g of phenylenediamine or a derivative thereof of formula II, 500-1000 ml of water, 60-90 ml of NaHSO₃ and 10-30 g of substituted benzaldehyde of formula III.

In a particular example, such a step comprises introducing into a 1000 ml balloon 25 g of 3,4-diaminobenzoic acid, 750 ml of water, 85 ml of NaHSO₃ 40% and 22,7 g of dihydroxybenzaldehyde.

In such an example, water is present at a concentration equal to 30 ml on a gram of substituted phenylenediamine of formula II or of 3,4-diaminobenzoic acid; NaHSO₃ 40% is present at a concentration equal to 3,4 ml on a gram of substituted phenylenediamine of formula II or of 3,4-diaminobenzoic acid.

Such a method comprises a step of agitating the mixture and a step of heating the mixture that take place simultaneously.

In particular, such steps take place at 78°C for 12 hours and/or at a temperature of over 70°C for a period comprised between 1 hour and 18 hours.

Such a method also comprises a step of cooling or allowing the reaction mixture to cool overnight.

At this point, there is a step of filtering and washing the solid obtained with hydrochloric acid for example with 500 ml of HCl 1N; possibly there is a further step of filtering the whole lot.

The method comprises a step of washing the solid on the filter with water and then with acetone; in particular such a step takes place with 300 ml of water and then with 300 ml of acetone.

Then follows a step of drying the compound at reduced pressure.

A product is obtained with purity HPLC 80%.

At this point, in order to increase the purity of the compound obtained, the method foresees to carry out an acid/base washing and grind the product or grind the product in a solvent or grind the product in methanol.

The product has a purity HPLC of 98%.

The total yield is 58%. Moreover, in the mother liquors there is still some product that could be recovered.

Such methods have overcome the following drawbacks, encountered using different method steps, in bulk processes for synthesising the compound of formula I.

Firstly, during the reaction step a mixture is obtained, the viscosity of which increases up to the end of the conversion. Such viscosity creates consequent filtration problems. Moreover, during the washing step with acidic water sulphur dioxide develops, which must consequently be treated.

The compound of formula I, moreover, is not very soluble in methanol, water, ethanol, acetone. Its solubility is, on the other hand, complete in basic water, for example NaOH, 1 N.

The methods described above have demonstrated better characteristics for synthesis at industrial level.

The compound object of the present invention was tested through different *in vitro* tests that complement one another, in order to evaluate its ability to counteract different radical species.

In particular, the DPPH test, based on the measurement of the reducing activity of anti-oxidant molecules against the radical DPPH (2,2-diphenyl-1-picrylhydrazyl), the FRAP (Ferric Reducing/Antioxidant Power) test, which measures the ability of an anti-oxidant to reduce the Fe³⁺ ions to Fe²⁺ ions in acid conditions and in the presence of TPTZ (2,4,6-tripyridyl-triazine) and PCL (Photochemiluminescence) analysis, which allows measurement of the inhibition by the anti-oxidants of photo-induced auto-oxidation of the luminol promoted by the superoxide anion, were used.

The compound object of the present invention has shown a significant increase in anti-oxidant activity with respect to 2-phenyl-1H-benzimidazole-5-sulfonic acid, which was taken as reference.

In table 1, in which the values are expressed in µmol Trolox/mmol compound, some examples are given.

**Table 1**

| | **DPPH** | **FRAP** | **PCL** |
|---|---|---|---|
| 2-phenyl-1H-benzoimidazole-5-sulfonic acid | 4,40 ± 0,03 | 0,79 ± 0,05 | < 0 |
| 2-(3,4-Dihydroxy-phenyl)-1H-benzoimidazole-5-sulfonic acid | 2145 ± 40 | 2313 ± 26 | 27855 ± 764 |
| 2-(3,4-Dihydroxy-phenyl)-1H-benzoimidazole-5-carboxylic acid | 1772 ± 75 | 1803 ± 22 | 22014 ± 360 |
| 2-(3,4-Dihydroxy-phenyl)-1H-benzimidazole | 1975 ± 15 | 2523 ± 29 | 27442 ± 329 |
| 2-(2,4-Dihydroxy-phenyl)-1H-benzimidazole | 16,5 ± 0,5 | 37,3 ± 0,3 | 198 ± 3 |
| 2-(2,4-Dihydroxy-phenyl)-1H-benzoimidazole-5-carboxylic acid | 32,2 ± 2,1 | 31,6 ± 1,7 | 160 ± 4 |
| 2-(4-Hydroxy-phenyl)-1H-benzoimidazole-5-carboxylic acid | 1,19 ± 0,02 | 2,62 ± 0,06 | 7 ± 0,3 |

Moreover, the compound object of the present invention recorded a significant increase in the maximum wavelength absorbable (X max) with respect to 2-phenyl-1H-benzimidazole-5-sulfonic acid, which has been taken as reference.

Some examples are given in table 2, in which the λ max were measured at pH comprised between 7,4 and 8 in a phosphate buffer.

**Table 2**

| | **λ max (nm)** |
|---|---|
| 2-phenyl-1H-benzimidazole-5-sulfonic acid | 302,5 |
| 2-(3,4-Dihydroxy-phenyl)-1H-benzoimidazole-5-sulfonic acid | 313,5 |
| 2-(3,4-Dihydroxy-phenyl)-1H-benzoimidazole-5-carboxylic acid | 317 |
| 2-(3,4-Dihydroxy-phenyl)-1H-benzimidazole | 311 |
| 2-(2,4-Dihydroxy-phenyl)-1H-benzimidazole | 315,5 |
| 2-(2,4-Dihydroxy-phenyl)-1H-benzoimidazole-5-carboxylic acid | 321,5 |
| 2-(4-Hydroxy-phenyl)-1H-benzoimidazole-5-carboxylic acid | 312,5 |

Considering the maximum absorption values in the UV region and the anti-oxidant activity values, the compound object of the present invention possesses water-soluble, UVA and UVB ultraviolet radiation filtering and anti-oxidant properties.

The compound according to the present invention is therefore recommended for use as UV filter and as agent to counteract free radicals.

According to a further version, the present invention concerns a pharmaceutical or cosmetic formulation or a medical device comprising the compound of formula I and/or its pharmaceutically acceptable salts.

The pharmaceutical or cosmetic formulation or the medical device of the present invention, as well as offering UVB protection and carrying out an anti-oxidant action, are able to widen the UV coverage spectrum also in the UVA range.

In such a pharmaceutical or cosmetic formulation or the medical device the compound according to the present invention acts as a UV filter and as contrasting agent of free radicals.

In such a pharmaceutical or cosmetic formulation or the medical device the compound according to the present invention is suitable for being used as UV filter and as contrasting agent of free radicals.

This has been demonstrated by comparing the SPF values, the ratio between UVA absorbance and UVB absorbance (UVA/UVB ratio) and the critical wavelength (critical X) of standard formulations each containing a compound object of the invention, in a concentration equal to 3%.

Determining the UVA/UVB ratio is a parameter that is mostly independent from the SPF value that provides a clear indication of the protective power of the solar product against UVA rays. According to the Boots scale, when the value of such a ratio is comprised between 0.6 and 0.8 the product offers a high UVA protection, when the value is greater than 0.8 the UVA protection is considered to be its maximum.

The critical λ is an important instrumental parameter for evaluating the protective power of the solar product against UVA rays: when the critical λ is greater than 370 nm the product offers protection over a wide spectrum. Determining the solar protection parameters was carried out through *in vitro* tests that foresee the use of spectrophotometric techniques, according to the international protocol Diffey and Robson (B.L. Diffey, J. Robson, J. Soc. Cosm. Chem., 1989, 40: 127-133).

As well as 2-phenyl-1H-benzimidazole-5-sulfonic acid, as reference product with which to compare the data a commercial filter (Neo Heliopan® AP) was also used, known to be a water-soluble exclusively UVA filter with critical λ greater than 370 nm. The results of some standard formulations containing a compound according to the present invention are given as an example in table 3.

**Table 3**

| | **SPF** | **UVA/UVB** | **λ critical nm** |
|---|---|---|---|
| 2-phenyl-1H-benzimidazole-5-sulfonic acid | 6,33 ± 1,24 | 0,31 ± 0,06 | 333 |
| Neo Heliopan® AP | 4,59 ± 0,37 | 2,46 ± 0,12 | 371 |
| 2-(3,4-Dihydroxy-phenyl)-1H-benzoimidazole-5-sulfonic acid | 13,36 ± 2,20 | 0,88 ± 0,04 | 358,5 |
| 2-(3,4-Dihydroxy-phenyl)-1H-benzoimidazole-5-carboxylic acid | 9,82 ± 1,30 | 1,21 ± 0,04 | 369,5 |
| 2-(3,4-Dihydroxy-phenyl)-1H-benzimidazole | 7,07 ± 1,36 | 0,93 ± 0, 02 | 367,5 |
| 2-(2,4-Dihydroxy-phenyl)-1H-benzimidazole | 4,85 ± 0,71 | 1,31 ± 0,04 | 379,5 |
| 2-(2,4-Dihydroxy-phenyl)-1H-benzoimidazole-5-carboxylic acid | 4,70 ± 0,62 | 1,48 ± 0,03 | 369,5 |
| 2-(4-Hydroxy-phenyl)-1H-benzoimidazole-5-carboxylic acid | 8,42 ± 1,55 | 0,89 ± 0,05 | 350 |

All of the formulations containing a compound of formula I, as described in the present invention, recorded an increase in the UVA/UVB ratio above 0.8 that indicates the maximum UVA protection in the Boots scale. Moreover, for the same formulations there is an increase in the critical X, such as to give the product a wide spectrum of UV protection. We then proceeded to measure the anti-oxidant activity through PCL analysis of the standard formulations containing a compound according to the present invention at the concentration of 3%. Such an anti-oxidant activity was measured in order to compare it with the reference molecules. Some examples are given in table 4.

**Table 4**

| | **µmol Trolox/g cosmetic formulation** |
|---|---|
| 2-phenyl-1H-benzimidazole-5-sulfonic acid | 0 |
| Neo Heliopan® AP | 0 |
| 2-(3,4-Dihydroxy-phenyl)-1H-benzoimidazole-5-sulfonic acid | 725 ± 3 |
| 2-(3,4-Dihydroxy-phenyl)-1H-benzoimidazole-5-carboxylic acid | 383 ± 18 |
| 2-(3,4-Dihydroxy-phenyl)-1H-benzimidazole | 760 ± 5 |
| 2-(2,4-Dihydroxy-phenyl)-1H-benzimidazole | 8,66 ± 0,04 |
| 2-(2,4-Dihydroxy-phenyl)-1H-benzoimidazole-5-carboxylic acid | 4, 97 ± 0,17 |
| 2-(4-Hydroxy-phenyl)-1H-benzoimidazole-5-carboxylic acid | 0,16 ± 0,02 |

In a version of the invention, the pharmaceutical or cosmetic formulation or the medical device according to the present invention comprises, in addition to the compound in question, one or more additional substances with UV filter properties, both for UVA and UVB, of organic and/or inorganic nature, in order to improve the protective action against UV rays.

Therefore, the pharmaceutical or cosmetic formulation or the medical device can comprise such additional substances without for this reason departing from the scope of protection of the present invention.

In general, all UV filters available on the market are suitable for combination with the compounds of formula I object of the present invention, preferring in particular the association with UV filters the safety of use of which has been demonstrated.

The additional substances of organic nature comprise at least one of the following substances:
- derivatives of camphor, like 3-(4'methylbenzylidene)-dl-camphor, e.g. Eusolex® 6300, 3-benzylidenecamphor, e.g. Mexoryl® S, polymers of N-{(2 and 4)-[(2-oxoborn-3-ylidene)-methyl]benzyl}-acrylamide, e.g. Mexoryl® SW, N,N,N,trimethyl-4-(2-oxoborn-3-ylidenemethyl)-anilinium methylsulfate, e.g. Mexoryl® SK, and/or α-(2-oxoborn-3-ylidene)toluene-4-sulfonic acid, e.g. Mexoryl® SL; and/or
- derivatives of benzoyl methane and/or dibenzoyl methane, such as 1-(4-tert-butylphenyl)-3-(4-methoxyphenyl)propane-1,3-dione, e.g. Eusolex® 9020, and/or 4-isopropyldibenzoylmethane; and/or
- benzophenones, such as 2-hydroxy-4-methoxybenzophenone, e.g. Eusolex® 4360, and/or 2-hydroxy-4¬methoxybenzophenone-5-sulfonic acid and/or its sodium salt, e.g. Uvinul® MS-40; and/or
- esters of methoxycinnamic acid, such as octyl methoxycinnamate, e.g. Eusolex® 2292, and/or isoamyl-4-methoxycinnamate, e.g. Neo Heliopan® and 1000; and/or
- salicylate derivative, such as 2-ethylhexyl salicylate, e.g. Eusolex® OS, 4-isopropylbenzyl salicylate, e.g. Megasol® and/or 3,3,5-trimethyl-cyclohexyl salicylate, e.g. Eusolex® HMS; and/or
- 4-aminobenzoic acid and/or its derivatives, such as 4-aminobenzoic acid, 2-ethylhexyl-4-(dimethylamino)benzoate, e.g. Eusolex® 600 and/or ethoxylated ethyl 4-aminobenzoate, e.g. Uvinul® P25; and/or
- benzimidazole derivatives, such as 2-phenylbenzimidazole-5-sulfonic acid, its potassium, sodium, lithium, ammonium and/or triethanolamine salts, e.g. Eusolex® 232, 2,2'-(1,4-phenylene)bis(1H-benzimidazole-4,6-disulfonic acid monosodium salt) and/or 2,2'-(1,4-phenylene)bis(1H-benzimidazole-5-sulfonic acid) and its potassium, sodium, and triethanolamine salts; and/or
- further substances, such as 2-ethylhexyl-2-cyano-3,3-diphenylacrylate, e.g. Eusolex® OCR, 3,3-(1,4-phenylenedimethylene)bis(7,7-dimethyl-2-oxobicyclo[2.2.1]hept-1-ylmethane sulfonic acid and/or its salts, e.g. Mexoryl® SX, 2,4,6-trianilino-(p-carbo-2'-ethylhexyl-1'-oxy)-1,3,5-triazine, e.g. Uvinul® T 150, 2-(2H-benzo-triazole-2-yl)-4-methyl-6-(2-methyl-3-(1,3,3,3-tetramethyl-1-(trimethylsilyloxy)disiloxanyl)propyl)phenol, e.g. Silatriazole® and/or similar substances.

The additional substances of inorganic nature comprise at least one of the substances such as the UV filters of the titanium dioxide group, like, for example, coated titanium dioxide, for example Eusolex ® T-2000, Eusolex ® T-AQUA, zinc oxides, for example Sachtotec ®, iron and/or cerium oxides and/or similar substances.

The listed compounds must be considered as examples, since it is also possible to use other UV filters.

The pharmaceutical or cosmetic formulation or the medical device according to the present invention comprises a percentage of compound of formula I and/or of other additional substances of organic nature equal to 0,5-20% by weight of pharmaceutical or cosmetic formulation or of the medical device, preferably equal to 1-15% by weight for each single substance.

Overall, the compound of formula I and the other additional substances of organic nature correspond to up to 40% by weight of pharmaceutical or cosmetic formulation or of medical device and preferably correspond to from 5 to 25% by weight of formulation.

The pharmaceutical or cosmetic formulation or the medical device according to the present invention comprises a percentage of compound of formula I and/or of other additional substances of inorganic nature equal to 0,5-20% by weight of pharmaceutical or cosmetic formulation or of medical device, preferably equal to 2-10% by weight for each single substance.

Different organic or inorganic UV filters can be used simultaneously in pharmaceutical or cosmetic formulations or in medical devices in the presence of at least one compound of formula I and in this case these can be used practically in any ratio with respect to each other.

In a further version of the invention, the protective action against oxidising stress and against the effect of the free radicals conferred by pharmaceutical or cosmetic formulations or the medical devices in accordance with the present invention can be further improved by inserting at least one other compound, or mixtures of compounds, with anti-oxidant properties.

For example, it is possible to use amino acids such as glycine, histidine, tyrosine, tryptophan and/or derivative products, and/or imidazoles, such as urocanic acid and/or derivatives, and/or peptides, such as D,L-carnosine, D-carnosine, L-carnosine and/or derivative products for example anserine, and/or carotenoids and/or carotenes, such as α-carotene, β-carotene, lycopene and/or derivatives, and/or chlorogenic acid and/or derivatives, and/or lipoic acid and/or derivative products for example dihydrolipoic acid, and/or thiols such as thioredoxin, glutathione, cysteine, cystine, cystamine and/or their glycoxyl, N-acetyl, methyl, ethyl, propylene, amyl, butyl, and lauryl, palmitic, oleic, γ-linoleic esters, esters of cholesterol and/or of glycerine, and/or the relative salts, and/or dilaurylthiodipropionate, and/or distearylthiodipropionate, and/or thiodipropionic acid and/or their derivatives such as esters, ethers, peptides, lipids, nucleotides, nucleosides and/or salts and/or sulfoximines such as buthionine-sulfoximine, sulfoximine homocysteine, buthionine-sulfone, penta-, hexa- and/or - heptathionine sulfoximine, in very low tolerated doses such as from picomols to micromols per kilogram, and/or chelating agents of metals such as α-hydroxy fatty acids, palmitic acid, phytic acid, lactoferrin, and/or α-hydroxy acids such as citric acid, lactic acid, malic acid, and/or humic acids, and/or biliary acids, and/or biliary extracts, and/or bilirubin, and/or biliverdin, and/or EDTA, and/or EGTA and/or their derivatives, and/or unsaturated fatty acids and/or their derivatives, and/or vitamin C and/or derivatives such as ascorbyl palmitate, magnesium ascorbyl phosfate, ascorbyl acetate, tocopherols and/or tocotrienols and/or their derivatives such as tocopheryl acetate, and/or vitamin A and/or derivatives such as vitamin A palmitate, and/or benzoin resin, and/or rutin and/or its derivatives and/or salts, and/or ferulic acid, and/or furfurylidineglucitol, and/or carosine, and/or butylhydroxy-toluene, and/or butylhydroxyanisole, and/or nordihydroguaiaretic acid, and/or trihydroxybutyrophenone, and/or quercetin, and/or uric acid and/or derivatives, and/or mannose and/or derivatives, and/or zinc and/or derivatives such as ZnO, ZnSO₄, and/or selenium and/or derivatives such as selenium methionine, and/or stilbenes and/or derivatives such as stilbene oxide, trans-stilbene oxide) and/or similar substances.

Other substances or mixtures with proven anti-oxidant activity are equally suitable for use in pharmaceutical or cosmetic formulations or medical devices according to the invention, like for example mixtures that comprise, as active ingredients, natural biophenols from plants, vegetable extracts titrated in polyphenols, mixtures of natural tocopherols and/or tocotrienols.

The formulations according to the invention can also comprise vitamins, like for example vitamin A and derivatives, for example vitamin A propionate, vitamin A palmitate, vitamin A acetate, retinol, vitamin B, thiamine and thiamine hydrochloride (vitamin B1) riboflavin (vitamin B2), nicotinamide, vitamin C (ascorbic acid), vitamin D, ergocalciferol (vitamin D2), vitamin E, DL-a-tocoferol, tocoferol acetate, tocoferol hydrogen succinate, vitamin K1, vitamin P, nicotinic acid (niacin), pyridoxine, pyridoxal, pyridoxamine (vitamin B6), pantotenic acid, biotin, folic acid, cobalamin (vitamin B12).

The compounds of formula I object of the present invention can be incorporated in pharmaceutical or cosmetic formulations or medical devices in the usual way. Suitable formulations are those for external use, like creams, lotions, gels or solutions that can be sprayed on the skin. Regarding this, when the preparation of the formulation comprises at least one oily phase and at least one aqueous phase, the compounds described in the invention are preferably inserted in the at least one aqueous phase.

The pharmaceutical or cosmetic formulation or the medical device according to the invention can be present in at least one form selected from: solutions, suspensions, emulsions, PIT emulsions, pastes, ointments, gels, creams, lotions, powders, soaps, hygiene products containing surfactants, oils, aerosols and sprays. Further examples of forms of application are sticks, shampoos and shower products.

Any carrier, auxiliary ingredient and/or further active ingredient can be added to the formulation.

In particular, auxiliary ingredients include the substances belonging to the classes of preservatives, anti-oxidants, stabilizers, solubilizers, vitamins, dyes and/or pigments, fragrances and perfumes, thickeners, emollients, hydraters, surfactants, emulsifiers, anti-foamers, waxes, lanolin, propellants and/or other ingredients generally used in pharmaceutical and/or cosmetic products.

Carriers suitable for the preparation of pomades, pastes, creams and gels can comprise ingredients normally used in the field of pharmaceuticals and/or cosmetics, like for example animal and vegetable fats, waxes, paraffins, starch, tragacanth, derivatives of cellulose, glycols, silicones, bentonite, silica, talc, zinc oxide or mixtures of such substances.

Powders and sprays can comprise known carriers, like for example lactose, talc, silica, aluminium hydroxide, calcium silicate, polyamide powder or mixtures of these substances. The sprays can also comprise commonly-used propellants, like hydrofluorocarbons, hydrocarbons such as propane, butane, isobutane, isopentane, dimethyl ether or compressed gases, for example nitrogen, carbon dioxide.

Solutions and emulsions can comprise commonly-used carriers, like solvents, emulsifiers, solubilizers, glycols, vegetable, mineral and synthesis oils, glycerol, esters of fatty acids, waxes or mixtures of these substances.

The suspensions can comprise commonly-used carriers like liquid dilutants, for example water, ethanol or propylene glycol; suspension agents, for example ethoxylated alcohols, esters of polyoxyethylene sorbitol, esters of polyoxyethylene sorbitan, microcrystalline cellulose, aluminium metahydroxide, bentonite, agar, tragacanth gum or mixtures of these substances.

The soaps can comprise commonly-used carriers, like salts of alkaline metals of fatty acids, salts of mono ester fatty acids, isothionates, lanolin, fatty alcohols, vegetable oils, vegetable extracts, glycerine, sugars or mixtures of these substances.

The hygiene products containing surfactants can comprise common ingredients, like salts of sulfated fatty alcohols, ethers of sulfated fatty alcohols, sulfosuccinic monoesters, isothionates, imidazole derivatives, methyl taurates, sarcosinates, amides of sulfated ether fatty acids, alkylamidobetaine, fatty alcohols, glycerides of fatty acids, diethanol amides of fatty acids, vegetable and synthetic oils, derivatives of lanolin, esters of fatty acids with ethoxylated glycerol or mixtures of such substances.

Oils for face and body can comprise commonly-used carriers, like synthetic oils, esters of fatty acids, fatty alcohols, silicone oils, natural oils, for example vegetable oils and extracts of oily plants, mineral oils, lanolin oils or mixtures of such substances.

Applications suitable for the formulations in accordance with the present invention also comprise the typical forms such as lipsticks, lip balm sticks, mascara, eyeliner, eye shadow, blush, powders, emulsions and waxes for make-up, skin protection products, like for example solar, pre-sun and after-sun products, products for protection from atmospheric agents.

All of the ingredients or the components that can be used in cosmetic formulations together with the compounds object of the invention are known and available on the market or can be synthesised with known processes.

The pharmaceutical or cosmetic formulation or the medical device in accordance with the invention are particularly suitable for protecting human skin against the harmful action of UV rays from sunlight, simultaneously offering protection for the skin itself against early ageing processes triggered by oxidising stress phenomena, i.e. against the damage caused by free radicals, which form through the action of solar radiation, of heat or of other factors such as pollution, food, atmospheric agents.

The pharmaceutical or cosmetic formulation or the medical device object of the invention can also be used to protect hair from photochemical damage, in order to avoid variations in colour tone, decolouration or mechanical damage. In this case, suitable formulations are in the form of shampoos, lotions, gels or emulsions for rinsing, products to be applied before or after shampoo, before or after colouration or decolouration or before or after a perm. It is also possible to use a formulation in the form of lotion, gel, spray for hair styling or hair care.

All pharmaceutical or cosmetic formulations or medical devices in accordance with the present invention can be prepared with techniques that are well-known to the man skilled in the art.

In order to further illustrate the present invention, some examples are given hereafter, for illustrative and not limiting purposes of the invention, which describe the preparation of some compounds of formula I and some possible ways to make a formulation that contains them, in accordance with what has been described up to now.

### EXAMPLE 1

### Preparation of 2-(3,4-dihydroxy-phenyl)-1H-benzoimidazole-5-sulfonic acid

In 5 ml of ethanol 100 mg of 3,4-diamine-benzenesulfonic acid sulfate salt (0.35 mmol), 48 mg of 3,4 dihydroxybenzaldehyde (0.35 mmol) and 0.7 ml of sodium bisulfite solution 1N (0.7 mmol) were mixed. The reaction mixture was placed at 80°C for 24 hours. After having evaporated the solvents, the raw product was treated with 5 ml of solution 5M of HCl. The precipitate was then filtered and the product recrystallized from methanol. The compound showed a maximum absorption in the UV region at 313 nm.

All of the compounds object of the invention were prepared with analogous procedure from suitably substituted reactants.

### EXAMPLE 2

**Standard formulation 3% prepared at pH 7,2-7,6**

| **Ingredients** | **% p/p** |
|---|---|
| **PHASE I** | **58.5** |
| Water | 52.8 |
| Glycerine | 5.0 |
| Phenoxyethanol, Methylparaben, Ethylparaben, Butylparaben, Propylparaben, Isobutylparaben | 0.7 |

| **PHASE II** | **20.5** |
|---|---|
| Cetyl alcohol, Glyceryl stearate, PEG-75 stearate, Ceteth-20, Steareth-20 | 6.0 |
| Cetyl alcohol | 2.0 |
| Dimethicone | 0.5 |
| C12-15 Alkyl Benzoate | 6.0 |
| Cocoglycerides | 6.0 |

| **PHASE III** | **21.0** |
|---|---|
| Solar filter (2-phenyl-1H-benzimidazole-5-sulfonic acid or Neo Heliopan AP or compound of formula I) | 3.0 |
| Water | 15.0 |
| Base (L-Arginine or NaOH solutione 10%) | 6.0 |
| **TOTAL** | **100.0** |

### EXAMPLE 3

**Solar spray (O/W, oil in water)**

| **Ingredients** | **% p/p** |
|---|---|
| **PHASE I** | **20.8** |
| Ethylhexyl Methoxycinnamate | 7.5 |
| Homosalate | 5.0 |
| Dimethicone | 1.0 |
| Steareth 2 | 0.4 |
| Steareth 10 | 0.8 |
| Acrylates/C10-30 Alkyl Acrylate Crosspolymer | 0.2 |
| Propylene Glycol Isoceteth-3 Acetate | 5.0 |
| Synthetic wax | 0.8 |
| Oxynex^{®} K | 0.1 |

| **PHASE II** | **79.2** |
|---|---|
| Compound of formula I | 3.0 |
| Water | 54.5 |
| L-Arginine | 6.0 |
| Propylene Glycol | 15.0 |
| Preservatives | 0.7 |
| **TOTAL** | **100.0** |

### EXAMPLE 4

**Solar emulsion (W/O, water in oil)**

| **Ingredients** | **% p/p** |
|---|---|
| **PHASE I** | **32.0** |
| Ethylhexyl Methoxycinnamate | 3.0 |
| Benzophenone-3 | 2.0 |
| Dimethicone | 1.0 |
| Hydrogenated Castor Oil | 1.0 |
| Cera Alba | 2.0 |
| Oleyl Erucate | 6.0 |
| Decyl Oleate | 6.0 |
| Dicapryl Ether | 5.0 |
| Dehymuls^{®} and | 6.0 |

| **PHASE II** | **68.0** |
|---|---|
| Compound of formula I | 4.0 |
| Water | 52.3 |
| Glycerine | 5.0 |
| NaOH 10% | 6.0 |
| Preservatives | 0.7 |
| **TOTAL** | **100.0** |

## Claims

1. Compound for use as a filter for the UVA and UVB ultraviolet radiation and as antioxidant agent to contrast free radicals, with 2-phenyl benzimidazolic structure of formula I and/or a pharmaceutically acceptable salt thereof, wherein said compound of formula I is water-soluble and corresponds to: 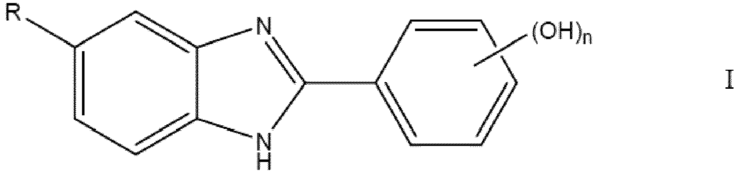 wherein n is 1, 2 or 3 and wherein R is a carboxyl radical (-COOH) or a sulphonic radical (-SO₃H).

2. Compound for use according to claim 1, wherein said pharmaceutically acceptable salt is a salt of alkali metals, of sodium or of potassium, or a salt of ammonium, of triethanolamine or of the corresponding carboxyl or sulphonic acids.

3. Pharmaceutical or cosmetic formulation or medical device in the form of an UVA and UVB filter and of an antioxidant agent suitable for contrasting free radicals comprising at least a compound with 2-phenyl benzimidazolic structure of formula I or a pharmaceutically acceptable salt thereof, according to any of claims 1-2, wherein said pharmaceutical or cosmetic formulation or medical device comprises at least one auxiliary ingredient selected among: preservatives, antioxidants, stabilizers, solubilizers, vitamins, colorants and/or pigments, fragrances and/or perfumes, thickeners, emollients, moisturizers, surfactants, emulsifiers, defoamers, waxes, lanolin, propellants.

4. Pharmaceutical or cosmetic formulation or medical device according to claim 3, comprising additional substances with UV filter properties, both UVA or UVB, of organic and/or inorganic nature.

5. Pharmaceutical or cosmetic formulation or medical device according to claim 4, wherein said additional substances of organic nature comprise at least one of the following substances:
3-(4'methylbenzylidene)-dl-camphor, 3-benzylidenecamphor, polymers of N-{(2 and 4)-[(2-oxoborn-3-ylidene)-methyl]benzyl}-acrylamide, N,N,N,trimethyl-4-(2-oxoborn-3-ylidenemethyl)-anilinium methylsulfate and/or α-(2-oxoborn-3-ylidene)toluene-4-sulfonic acid, and/or any other camphor derivative suitable for the object; and/or
1-(4-tert-butylphenyl)-3-(4-methoxyphenyl)propane-1,3-dione, and/or 4-isopropyldibenzoylmethane, and/or any other benzoylmethane and/or dibenzoylmethane derivative suitable for the object; and/or
2-hydroxy-4-methoxybenzophenone, and/or 2-hydroxy-4-methoxybenzophenone-5-sulphonic acid and/or sodium salt thereof, and/or any other benzophenone suitable for the object; and/or
octyl methoxycinnamate, and/or isoamyl-4-methoxycinnamate, and/or any other ester of methoxycinnamate acid suitable for the object; and/or 2-ethylhexyl salicylate, 4-isopropylbenzyl salicylate, and/or 3,3,5-trimethyl-cyclohexyl salicylate, and/or any other salicylate derivative suitable for the object; and/or
4-aminobenzoic acid and/or 4-aminobenzoic acid, 2-ethylhexyl-4-(dimethyl-amino)benzoate, and/or ethoxylated ethyl 4-aminobenzoate, and/or any other 4-aminobenzoic acid derivative suitable for the object; and/or
2-acid phenylbenzimidazole-5-sulphonic, potassium, sodium, lithium, ammonium and/or triethanolamine salts thereof, monosodium salt of 2,2'-(1,4-phenylene)bis(1Hbenzimidazole-4,6-disulfonic) and/or 2,2'-(1,4-phenylene)bis(1H-benzimidazole-5-sulphonic acid) and potassium, sodium and triethanolamine salts thereof, and/or any other benzimidazole derivative suitable for the object; and/or
2-ethylhexyl-2-cyano-3,3-diphenylacrylate, 3,3-(1,4-phenylenedimethylene)bis(7,7-dimethyl-2-xobicyclo[2.2.1]hept-1-ylmethane sulphonic acid and/or salts thereof, 2,4,6-trianilino-(p-carbo-2'-ethylhexyl-1'-oxy)-1,3,5-triazine, 2-(2H-benzotriazole-2-yl)-4-methyl-6-(2-methyl-3-(1,3,3,3-tetramethyl-1-(trimethylsilyloxy)disiloxanyl)propyl)phenol, and/or similar substances.

6. Pharmaceutical or cosmetic formulation or medical device according to claim 4, wherein said additional substances of inorganic nature comprise at least one of the following UV filters: UV filters of the titanium dioxide group, and/or coated titanium dioxide and/or zinc oxides and/or iron oxides and/or cerium oxides and/or similar substances.

7. Pharmaceutical or cosmetic formulation or medical device according to any one of claims 3-6, wherein said compound of formula I and/or said additional substances of organic nature and/or of inorganic nature are provided in a percentage equal to 0,5-20% by weight of pharmaceutical or cosmetic formulation or medical device, for each single substance.

8. Pharmaceutical or cosmetic formulation or medical device according to any one of claims 3-6, wherein said compound of formula I and/or said additional substances of organic nature are provided in a percentage equal to 1-15% by weight of pharmaceutical or cosmetic formulation or medical device, for each single substance.

9. Pharmaceutical or cosmetic formulation or medical device according to any one of claims 3-6, wherein said compound of formula I and/or said additional substances of inorganic nature are provided in a percentage equal to 2-10% by weight of pharmaceutical or cosmetic formulation or medical device, for each single substance.

10. Pharmaceutical or cosmetic formulation or medical device according to any one of claims 3-9, comprising up to 40% by weight of pharmaceutical or cosmetic formulation or medical device of said compound of formula I and/or of said additional substances of organic nature.

11. Pharmaceutical or cosmetic formulation or medical device according to any one of claims 3-9, comprising 5 to 25% by weight of pharmaceutical or cosmetic formulation or medical device of said compound of formula I and/or of said additional substances of organic nature.

12. Pharmaceutical or cosmetic formulation or medical device according to any one of claims 3-11, comprising at least one compound or a mixture of compounds with antioxidant properties, selected among: amino acids and/or derivative products thereof and/or imidazoles and/or derivatives thereof and/or peptides and/or derivative products thereof and/or carotenoids and/or carotenes and/or derivatives thereof and/or chlorogenic acid and/or derivatives thereof and/or lipoic acid and or derivative products thereof and/or thiols and/or glycosyic, N-acetyls, methyls, ethyls, propyls, amyls, butyls, lauryls, palmitics, oleics, γ-linoleics esters thereof, of cholesterol and/or of glycerine and/or respective salts and/or dilauryl thiodipropionate and/or distearyl thiodipropionate and/or thiodipropionate acid and/or derivatives thereof and/or sulfoximines and/or metals chelating agents and/or α-hydroxy acids and/or humic acids and/or bile acids and/or bile extracts and/or bilirubin and/or biliverdin and/or EDTA and/or EGTA and/or derivatives thereof and/or unsatured fatty acids and/or derivatives thereof and/or vitamin C and/or derivatives thereof and/or tocopherols and/or tocotrienols and/or derivatives thereof and/or vitamin A and/or derivatives thereof and/or benzoino resin and/or rutin and/or derivatives thereof and/or salts and/or ferulic acid and/or furfurylidineglucitol and/or carnosine and/or butylhydroxy-toluene and/or butylhydroxyanisole and/or nordihydroguaiaretic acid and/or trihydroxybutyrophenone and/or quercetin and/or uric acid and/or derivatives thereof and/or mannose and/or derivatives thereof and/or zinc and/or derivatives thereof and/or selenium and/or derivatives thereof and/or stilbenes and/or derivatives thereof and/or natural biophenols from plants and/or vegetal extracts titrated in polyphenols and/or natural tocopherols and/or tocotrienols mixtures, and/or retinol, and/or vitamin B and/or thiamine and/or thiamine hydrochloride and/or riboflavin and/or nicotinamide and/or vitamin C and/or vitamin D and/or ergocalciferol and/or vitamin E and/or DL-a-tocopherol and/or tocopherol acetate and/or tocopherol hydrogen succinate and/or vitamin K1 and/or vitamin P and/or nicotinic acid and/or pyridoxine and/or pyridoxal and/or pyridoxamine and/or pantothenic acid and/or biotin and/or folic acid and/or cobalamin and/or similar substances.

13. Pharmaceutical or cosmetic formulation or medical device according to any one of claims 3-12, in at least one form selected among the following: solution, suspension, emulsion, PIT emulsion, paste, ointment, gel, cream, lotion, powder, spray, soap, hygiene product containing surfactants, oil, aerosol, stick, shampoo and/or shower product, lip-sticks, lip-care sticks, rimmel, eyeliners, eye shadows, blushers, powders, make-up emulsions and/or waxes, skin protection products, such as sunscreen, pre sun and/or after sun, products, products for the protection against atmospheric agents, formulations suitable for hair styling and care such as shampoo, lotions, gel, spray and/or rinsing emulsions, products applied before or after shampoo, before or after coloring or bleaching and/or before or after perm.

14. Pharmaceutical or cosmetic formulation or medical device according to any one of claims 3-13, comprising at least one suitable carrier selected among: animal and/or vegetal fats, waxes, paraffins, starch, tragacanth, cellulose derivatives, glycoles, silicones, bentonite, silica, talc, zinc oxide and/or mixtures of such substances, lactose, talc, silica, aluminum hydroxide, calcium silicate, polyamide powder and/or mixtures of such substances, propellants such as hydrofluorocarbons, hydrocarbons, dimethyl ether and/or compressed gases, solvents, emulsifiers, solubilizers, glycoles, vegetal, mineral and/or synthetic oils, glycerol, fatty acids esters, waxes and/or mixtures of such substances, liquid diluents such as water, ethanol and/or propylene glycol, suspending agents, such as ethoxylated alcohols, esters of polyoxyethilene sorbitol, esters of polyoxyethilene sorbitan, microcrystalline cellulose, aluminum metahydroxide, bentonite, agar, tragacanth and/or mixtures of such substances, salts of alkali metals of fatty acids, salts of fatty acids mono-esters, isothionates, lanolin, fatty alcohols, vegetal oils, vegetal extracts, glycerin, sugars and/or mixtures of such substances, salts of sulfated fatty alcohols, ethers of sulfated fatty alcohols, monoester sulfosuccinates, isothionates, imidazoline derivatives, methyl taurates, sarcosinates, amides of fatty acids ether sulfates, alkylamido betaines, fatty alcohols, fatty acids glycerides, fatty acids diethanolamides, vegetal and synthetic oils, lanolin derivatives, fatty acids esters with glycerol ethoxylate and/or mixtures of such substances, synthetic oils, fatty acids esters, fatty alcohols, silicon oils, natural oils, mineral oils, lanolin oils and/or mixtures of such substances.

## Patentansprüche

1. Verbindung zur Verwendung als Filter für die UV-A-und UV-B-Ultraviolettstrahlung und als Antioxidationsmittel zum Entgegenwirken gegen freie Radikale mit 2-Phenyl-Benzimidazol-Struktur der Formel I und/oder einem pharmazeutisch verträglichen Salz davon, worin die besagte Verbindung der Formel I wasserlöslich ist und Folgendem entspricht: 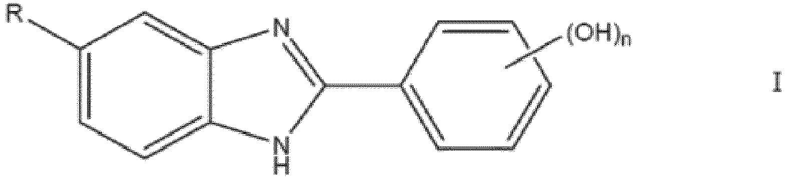 worin n 1, 2 oder 3 ist und worin R ein Carboxyl-Radikal (-COOH) oder ein Sulfon-Radikal (-SO₃H) ist.

2. Verbindung zur Verwendung nach Anspruch 1, worin das besagte pharmazeutisch verträgliche Salz ein Alkalimetallsalz von Natrium oder Kalium oder ein Ammoniumsalz, ein Triethanolaminsalz oder ein Salz der entsprechenden Carboxyl- oder Sulfonsäuren ist.

3. Pharmazeutische oder kosmetische Formulierung oder Medizinprodukt in Form eines UV-A- und UV-B-Filters und eines Antioxidationsmittels, das zum Entgegenwirken gegen freie Radikale geeignet ist, umfassend mindestens eine Verbindung mit 2-Phenyl-Benzimidazol-Struktur der Formel I oder ein pharmazeutisch verträgliches Salz davon nach irgendeinem der Ansprüche 1-2, worin die besagte pharmazeutische oder kosmetische Formulierung oder das Medizinprodukt mindestens einen Hilfsstoff umfasst, ausgewählt aus: Konservierungsmitteln, Antioxidantien, Stabilisatoren, Solubilisatoren, Vitaminen, Farbstoffen und/oder Pigmenten, Duftstoffen und/oder Parfüms, Verdickungsmitteln, Aufweichmitteln, Feuchthaltemitteln, oberflächenaktiven Mitteln, Emulgatoren, Entschäumern, Wachsen, Lanolin, Treibmitteln.

4. Pharmazeutische oder kosmetische Formulierung oder Medizinprodukt nach Anspruch 3, umfassend Zusatzstoffe mit UV-Filter-Eigenschaften, sowohl für UV-A als auch für UV-B, organischer oder anorganischer Natur.

5. Pharmazeutische oder kosmetische Formulierung oder Medizinprodukt nach Anspruch 4, worin die besagten Zusatzstoffe organischer Natur mindestens eine der folgenden Substanzen umfassen:
3-(4'-Methylbenzyliden)-DL-Campher, 3-Benzylidencampher, Polymere von N-{(2 und 4)-[(2-oxoborn-3-yliden)-methyl]benzyl}-acrylamid, N,N,N,Trimethyl-4-(2-Oxoborn-3-ylidenmethyl)-aniliniummethylsulfat und/oder alpha-(2-Oxoborn-3-yliden)toluen-4-sulfonsäure, und/oder jedes andere Campherderivat, das für den Zweck geeignet ist; und/oder 1-(4-tert-Butylphenyl)-3-(4-methoxyphenyl)propan-1, 3-dion, und/oder 4-Isopropyldibenzoylmethan, und/oder jedes andere Benzoylmethan und/oder Dibenzoylmethanderivat, das für den Zweck geeignet ist; und/oder
2-Hydroxy-4-methoxy-benzophenon, und/oder 2-Hydroxy-4-methoxy-benzophenon-5-sulfonsäure und/oder Natriumsalz davon, und/oder jedes andere Benzophenon, das für den Zweck geeignet ist; und/oder
Octylmethoxycinnamat, und/oder Isoamyl-4-methoxycinnamat, und/oder jedes andere Ester einer Methoxycinnamat-Säure, das für den Zweck geeignet ist; und/oder
2-Ethylhexylsalicylat, 4-Isopropylbenzylsalicylat, und/oder 3,3,5-Trimethylcyclohexyl-salicylat, und/oder jedes andere Salicylatderivat, das für den Zweck geeignet ist; und/oder
4-Aminobenzoesäure und/oder 4-Aminobenzoesäure, 2-Ethylhexyl-4-(dimethyl-amino)benzoat, und/oder ethoxyliertes Ethyl-4-aminobenzoat und/oder jedes andere 4-Aminobenzoesäurederivat, das für den Zweck geeignet ist; und/oder
2-Phenylbenzimidazol-5-sulfonsäure, Kalium, Natrium, Lithium, Ammonium und/oder Triethanolaminsalze davon, Mononatriumsalz von 2,2'-(1,4-Phenylen)bis(lHbenzimidazol-4,6-disulfon) und/oder 2,2'-(1,4-Phenylen)bis(1H-benzimidazol-5-sulfonsäure) und Kalium, Natrium und Triethanolaminsalze davon, und/oder jedes andere Benzimidazolderivat, das für den Zweck geeignet ist; und/oder
2-Ethylhexyl-2-cyan-3,3-diphenylacrylat, 3,3- (1,4-Phenylenedimethylen)bis(7,7-dimethyl-2-oxo-bicyclo [2.2.1]hept-1-ylmethan-sulfonsäure und/oder Salze davon, 2,4,6-Trianilin-(p-carbo-2'- ethylhexyl-1'-oxy)-1,3,5-triazin, 2-(2H-Benzotriazol-2-yl)-4-methyl-6-(2-methyl-3-(1,3,3,3-Tetramethyl-1-(trimethylsilyloxy)disiloxanyl)propyl)phenol, und/oder ähnliche Stoffe.

6. Pharmazeutische oder kosmetische Formulierung oder Medizinprodukt nach Anspruch 4, worin die besagten Zusatzstoffe anorganischer Natur mindestens einen der folgenden UV-Filter umfassen: UV-Filter der Titandioxid-Gruppe, und/oder beschichtetes Titandioxid und/oder Zinkoxide und/oder Eisenoxide und/oder Ceroxide und/oder ähnliche Stoffe.

7. Pharmazeutische oder kosmetische Formulierung oder Medizinprodukt nach irgendeinem der Ansprüche 3-6, worin die besagte Verbindung der Formel I und/oder die besagten Zusatzstoffe organischer Natur und/oder anorganischer Natur in einem Prozentsatz von gleich 0,5-20 Gewichts-% einer pharmazeutischen oder kosmetischen Formulierung oder eines Medizinproduktes für jeden einzelnen Stoff vorhanden sind.

8. Pharmazeutische oder kosmetische Formulierung oder Medizinprodukt nach irgendeinem der Ansprüche 3-6, worin die besagte Verbindung der Formel I und/oder der besagten Zusatzstoffe organischer Natur in einem Prozentsatz von gleich 1-15 Gewichts-% einer pharmazeutischen oder kosmetischen Formulierung oder eines Medizinproduktes für jeden einzelnen Stoff vorhanden sind.

9. Pharmazeutische oder kosmetische Formulierung oder Medizinprodukt nach irgendeinem der Ansprüche 3-6, worin die besagte Verbindung der Formel I und/oder der besagten Zusatzstoffe anorganischer Natur in einem Prozentsatz von gleich 2-10 Gewichts-% einer pharmazeutischen oder kosmetischen Formulierung oder eines Medizinproduktes für jeden einzelnen Stoff vorhanden sind.

10. Pharmazeutische oder kosmetische Formulierung oder Medizinprodukt nach irgendeinem der Ansprüche 3-9, umfassend bis zu 40 Gewichts-% einer pharmazeutischen oder kosmetischen Formulierung oder eines Medizinproduktes der besagten Verbindung der Formel I und/oder der besagten Zusatzstoffe organischer Natur.

11. Pharmazeutische oder kosmetische Formulierung oder Medizinprodukt nach irgendeinem der Ansprüche 3-9, umfassend 5 bis 25 Gewichts-% einer pharmazeutischen oder kosmetischen Formulierung oder eines Medizinproduktes der besagten Verbindung der Formel I und/oder der besagten Zusatzstoffe organischer Natur.

12. Pharmazeutische oder kosmetische Formulierung oder Medizinprodukt nach irgendeinem der Ansprüche 3-11, umfassend mindestens eine Verbindung oder ein Gemisch von Verbindungen mit antioxidativen Eigenschaften, ausgewählt aus: Aminosäuren und/oder Derivatprodukte davon und/oder Imidazole und Derivate davon und/oder Peptide und Derivatprodukte davon und/oder Carotenoide und/oder Carotine und/oder Derivate davon und/oder Chlorogensäure und/oder Derivate davon und/oder Liponsäure und/oder Derivatprodukte davon und/oder Thiole und/oder Glykosid-, N-Acetyl-, Methyl-, Ethyl-, Propyl-, Amyl-, Butyl-, Lauryl-, Palmitin-, Olein-, Gamma-Linol-Ester davon, von Cholesterin und/oder von Glyzerin und/oder entsprechende Salze und/oder Dilaurylthiodipropionat und/oder Distearylthiodipropionat und/oder Thiodipropionat-Säure und/oder Derivate davon und/oder Sulfoximine und/oder Metallchelatbildner und/oder Alpha-Hydroxysäuren und/oder Huminsäuren und/oder Gallensäuren und/oder Gallenextrakte und/oder Bilirubin und/oder Biliverdin und/oder EDTA und/oder EGTA und/oder Derivate davon und/oder ungesättigte Fettsäuren und/oder Derivate davon und/oder Vitamin C und/oder Derivate davon und/oder Tocopherole und/oder Tocotrienole und/oder Derivate davon und/oder Vitamin A und/oder Derivate davon und/oder Benzoinharz und/oder Rutin und/oder Derivate davon und/oder Salze und/oder Ferulsäure und/oder Furfurylidineglucitol und/oder Carnosin und/oder Butylhydroxytoluol und/oder Butylhydroxyanisol und/oder Nordihydroguajaretsäure und/oder Trihydroxybutyrophenon und/oder Quercitin und/oder Harnsäure und/oder Derivate davon und/oder Mannose und/oder Derivate davon und/oder Zink und/oder Derivate davon und/oder Selen und/oder Derivate davon und/oder Stilbene und/oder Derivate davon und/oder natürliche Biophenole von Pflanzen und/oder pflanzliche Extrakte titriert in Polyphenolen und/oder natürliche Tocopherole und/oder Tocotrienol-Gemische, und/oder Retinol, und/oder Vitamin B und/oder Thiamin und/oder Thiaminhydrochlorid und/oder Riboflavin und/oder Nicotinamid und/oder Vitamin C und/oder Vitamin D und/oder Ergocalciferol und/oder Vitamin E und/oder DLalpha-Tocopherol und/oder Tocopherolacetat und/oder Tocopherolhydrogensuccinat und/oder Vitamin K1 und/oder Vitamin P und/oder Nicotinsäure und/oder Pyridoxin und/oder Pyridoxal und/oder Pyridoxamin und/oder Pantothensäure und/oder Biotin und/oder Folsäure und/oder Cobalamin und/oder ähnliche Stoffe.

13. Pharmazeutische oder kosmetische Formulierung oder Medizinprodukt nach irgendeinem der Ansprüche 3-12, in mindestens einer Form, ausgewählt aus Folgendem: Lösung, Suspension, Emulsion, PIT-Emulsion, Paste, Salbe, Gel, Creme, Lotion, Pulver, Spray, Seife, tensidhaltiges Hygieneprodukt, Öl, Aerosol, Stift, Shampoo und/oder Duschprodukt, Lippenstifte, Lippenpflegestifte, Wimperntusche, Augenkonturenstifte, Lidschatten, Rouge, Puder, Schminkemulsionen und/oder - wachse, Hautschutzmittel wie Sonnenschutz-, Pre-Sun- und/oder After-Sun-Produkte, Produkte zum Schutz gegen atmosphärische Einflüsse, Formulierungen, die für das Haarstyling und die Haarpflege geeignet sind, wie Shampoo, Lotionen, Gel, Spray und/oder Spülemulsionen, Produkte, die vor und nach dem Shampoo, vor oder nach dem Färben oder Bleichen und/oder vor oder nach einer Dauerwelle aufzutragen sind.

14. Pharmazeutische oder kosmetische Formulierung oder Medizinprodukt nach irgendeinem der Ansprüche 3-13, umfassend mindestens einen geeigneten Träger, ausgewählt aus: tierischen oder pflanzlichen Fetten, Wachsen, Paraffinen, Stärke, Traganth, Cellulosederivaten, Glycolen, Silikonen, Bentonit, Kieselerde, Talk, Zinkoxid und/oder Gemischen derartiger Stoffe, Laktose, Talk, Kieselerde, Aluminiumhydroxid, Kalziumsilikat, Polyamidpulver und/oder Gemischen derartiger Stoffe, Treibmitteln wie Fluorkohlenwasserstoffe, Kohlenwasserstoffe, Dimethylether und/oder verdichtete Gase, Lösungsmitteln, Emulgatoren, Solubilisatoren, Glykolen, pflanzlichen, mineralischen und/oder synthetischen Ölen, Glyzerol, Fettsäureestern, Wachsen und/oder Gemischen derartiger Stoffe, flüssigen Verdünnungsmitteln wie Wasser, Ethanol und/oder Propylenglykol, Suspensionsmitteln, wie ethoxylierte Alkohole, Polyoxyethylensorbitolester, Polyoxyethylensorbitanester, mikrokristallinen Cellulosen, Aluminum-Metahydroxid, Bentonit, Agar, Traganth und/oder Gemischen derartiger Stoffe, Alkalimetallsalzen von Fettsäuren, Salzen von Fettsäuremonoestern, Isothionaten, Lanolin, Fettalkoholen, pflanzlichen Ölen, pflanzlichen Extrakten, Glyzerin, Zucker und/oder Gemischen derartiger Stoffe, Salzen sulfatierter Fettsäuren, Ethern sulfatierter Fettalkohole, Monoester-Sulfosuccinaten, Isothionaten, Imidazolinderivaten, Methyltauraten, Sarcosinaten, Amiden von Fettsäure-Ethersulfaten, Alkylamido-Betainen, Fettalkoholen, Fettsäureglyceriden, Fettsäurediethanolamiden, pflanzlichen und synthetischen Ölen, Lanolinderivaten, Fettsäureestern mit Glycerolethoxylat und/oder Gemischen derartiger Stoffe, synthetischen Ölen, Fettsäureestern, Fettalkoholen, Silikonölen, natürlichen Ölen, mineralischen Ölen, Lanolinölen und/oder Gemischen derartiger Stoffe.

## Revendications

1. Composé pour être utilisé comme filtre pour les rayons ultraviolets UVA et UVB et comme agent antioxydant pour s'opposer aux radicaux libres, ayant une structure 2-phénylbenzimidazolique de formule I et/ou un sel pharmaceutiquement acceptable de celui-ci, dans lequel ledit composé de formule I est soluble dans l'eau et correspond à : 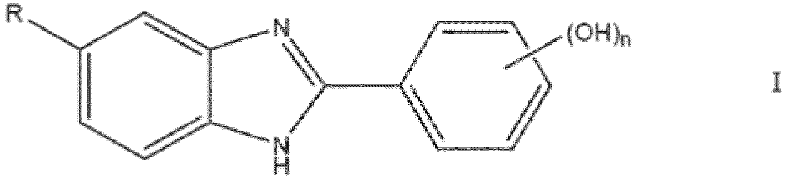 où n est 1, 2 ou 3 et où R représente un radical carboxyle (-C00H) ou un radical sulfonique (-SO₃H).

2. Composé pour l'utilisation selon la revendication 1, dans lequel ledit sel pharmaceutiquement acceptable est un sel de métaux alcalins, de sodium ou de potassium, ou un sel d'ammonium, de triéthanolamine ou des acides carboxyliques ou sulfoniques correspondants.

3. Formulation pharmaceutique ou cosmétique ou dispositif médical sous la forme d'un filtre UVA et UVB et d'un agent antioxydant approprié pour s'opposer aux radicaux libres comprenant au moins un composé ayant une structure 2-phénylbenzimidazolique de formule I ou un sel pharmaceutiquement acceptable de celui-ci, selon les revendications 1 ou 2, dans laquelle ou lequel ladite formulation pharmaceutique ou cosmétique ou ledit dispositif médical comprend au moins un ingrédient auxiliaire sélectionné parmi les suivants : conservateurs, antioxydants, stabilisateurs, agents solubilisants, vitamines, colorants et/ou pigments, fragrances et/ou parfums, épaississants, émollients, hydratants, surfactants, émulsifiants, antimousses, cires, lanoline, propulseurs.

4. Formulation pharmaceutique ou cosmétique ou dispositif médical selon la revendication 3, comprenant des substances additionnelles ayant des propriétés de filtre UV, à la fois UVA ou UVB, de nature organique et/ou inorganique.

5. Formulation pharmaceutique ou cosmétique ou dispositif médical selon la revendication 4, dans laquelle ou lequel lesdites substances additionnelles de nature organique comprennent au moins une des substances suivants :
3-(4'méthylbenzylidène)-dl-camphre, 3-benzylidène camphre, polymères de N-{(2 et 4)-[(oxo-2 bornylidène-3)-méthyl]benzyl}-acrylamide, Sulfate de méthyle de N,N,N-triméthyl [(oxo-2 bornylidène-3) méthyl]-4 anilinium et/ou acide alpha-(oxo-2 bornylidène-3)-toluène-4-sulfonique, et/ou tout autre dérivé de camphre approprié pour l'objet ; et/ou
(Tert-butyl-4 phénol) -1 (méthoxy-4 phényl)-3 propanedione-1,3, et/ou 4-isopropyldibenzoylméthane, et/ou tout autre dérivé de benzoylméthane et/ou de dibenzoylméthane approprié pour l'objet ; et/ou 2-hydroxy-4-méthoxybenzophénone, et/ou acide 2-hydroxy-4-méthoxybenzophénone-5-sulfonique et/ou son sel sodique, et/ou tout autre benzophénone approprié pour l'objet ; et/ou
octyl méthoxycinnamate, et/ou isoamyle-4-méthoxycinnamate, et/ou tout autre ester d'acide méthoxycinnamique approprié pour l'objet ; et/ou salicylate d'éthyle-2 hexyle, salicylate d'isopropyl-4 benzyle, et/ou salicylate de triméthyl-3,3,5 cyclohexyle, et/ou tout autre dérivé de salicylate approprié pour l'objet ;et/ou
acide 4-aminobenzoïque et/ou acide 4-aminobenzoïque, 4-(diméthylamino)benzoate de 2-éthylhexyle, et/ou 4-éthoxy aminobenzoate d'éthyle, et/ou tout autre dérivé de l'acide 4-aminobenzoïque approprié pour l'objet ; et/ou
acide 2-phénylbenzimidazole-5-sulfonique, son sels de potassium, sodium, lithium, ammonium et/ou triéthanolamine, sel monosodique d'acide 2,2'-(1,4-phénylène)bis(lHbenzimidazole-4, 6-disulfonique) et/ou acide 2,2' -(1,4-phénylène)bis(1H-benzimidazole-5-sulphonique) et son sels de potassium, sodium et triéthanolamine, et/ou tout autre dérivé de benzimidazole approprié pour l'objet ; et/ou
acrylate de 2-éthylhexyl-2-cyano-3,3-diphényle, acide sulfonique 3,3- (1,4-phénylènediméthylène)bis(7,7-diméthyle-2-oxobicyclo[2.2.1]hept-1-ylméthane et/ou leurs sels, 2,4,6-Trianilino-(P-carbo-2'-éthylhexyle-l'-oxi)-1,3,5-triazine, 2-(2H-benzotriazol-2-yl)-4-méthyl-6-(2-méthyl-3-(1,3,3,3-tétraméthyl-1-(triméthylsilyl)oxy)-disiloxanyl)propyl) phénol, et/ou autres substances similaires.

6. Formulation pharmaceutique ou cosmétique ou dispositif médical selon la revendication 4, dans laquelle ou lequel lesdites substances additionnelles de nature inorganique comprennent au moins un des filtres UV suivants : filtres UV du groupe dioxyde de titane et/ou dioxyde de titane revêtu et/ou oxydes de zinc et/ou oxydes de fer et/ou oxydes de cérium et/ou autres substances similaires.

7. Formulation pharmaceutique ou cosmétique ou dispositif médical selon l'une des revendications 3 à 6, dans laquelle ou lequel ledit composé de formule I et/ou lesdites substances additionnelles de nature organique et/ou de nature inorganique sont fournis dans un pourcentage égal à 0,5 à 20 % en poids de formulation pharmaceutique ou cosmétique ou dispositif médical, pour chacune des substances.

8. Formulation pharmaceutique ou cosmétique ou dispositif médical selon l'une des revendications 3 à 6, dans laquelle ou lequel ledit composé de formule I et/ou lesdites substances additionnelles de nature organique sont fournis dans un pourcentage égal à 1 à 15 % en poids de formulation pharmaceutique ou cosmétique ou dispositif médical, pour chacune des substances.

9. Formulation pharmaceutique ou cosmétique ou dispositif médical selon l'une des revendications 3 à 6, dans laquelle ou lequel ledit composé de formule I et/ou lesdites substances additionnelles de nature inorganique sont fournis dans un pourcentage égal à 2 à 10 % en poids de formulation pharmaceutique ou cosmétique ou dispositif médical, pour chacune des substances.

10. Formulation pharmaceutique ou cosmétique ou dispositif médical selon l'une des revendications 3 à 9, comprenant jusqu'à 40 % en poids de formulation pharmaceutique ou cosmétique ou dispositif médical dudit composé de formule I et/ou desdites substances additionnelles de nature organique.

11. Formulation pharmaceutique ou cosmétique ou dispositif médical selon l'une des revendications 3 à 9, comprenant 5 à 25% en poids de formulation pharmaceutique ou cosmétique ou dispositif médical dudit composé de formule I et/ou desdites substances additionnelles de nature organique.

12. Formulation pharmaceutique ou cosmétique ou dispositif médical selon l'une des revendications 3 à 11, comprenant au moins un composé ou un mélange de composés ayant des propriétés antioxydantes, sélectionné parmi les suivants : acides aminés et/ou leurs produits dérivés et/ou imidazoles et/ou leurs dérivés et/ou peptides et/ou leurs produits dérivés et/ou caroténoïdes et/ou carotènes et/ou leurs dérivés et/ou acide chlorogénique et/ou ses dérivés et/ou acide lipoïque et/ou ses produits dérivés et/ou thiols et/ou leurs esters glycosydique, de N-acétyles, méthyles, éthyles, propyles, amyles, butyles, lauryles, palmitiques, oléiques, γ-linoléiques, de cholestérol et/ou de glycérine et/ou leurs sels respectifs et/ou thiodipropionate de dilauryle et/ou thiodipropionate de distéaryle et/ou acides thiodipropionique et/ou leurs dérivés et/ou sulfoximines et/ou agents chélateurs métalliques et/ou acides α-hydroxy et/ou acides humiques et/ou acides biliaires et/ou extraits de bile et/ou bilirubine et/ou biliverdine et/ou EDTA et/ou EGTA et/ou leurs dérivés et/ou acides gras insaturés et/ou leurs dérivés et/ou vitamine C et/ou leurs dérivés et/ou tocophérols et/ou tocotriénols et/ou leurs dérivés et/ou vitamine A et/ou ses dérivés et/ou résine de benjoin et/ou rutine et/ou leurs dérivés et/ou sels et/ou acide férulique et/ou furfurylidineglucitol et/ou carnosine et/ou butylhydroxytoluène et/ou butylhydroxyanisole et/ou acide nor-dihydro-guaïarétique et/ou trihydroxybutyrophénone et/ou quercétine et/ou acide urique et/ou leurs dérivés et/ou mannose et/ou ses dérivés et/ou zinc et/ou ses dérivés et/ou sélénium et/ou ses dérivés et/ou stilbène et/ou ses dérivés et/ou biophénols naturels de plantes et/ou extraits végétaux titrés en polyphénols et/ou tocophérols naturels et/ou mélanges de tocotriénols, et/ou rétinol, et/ou vitamine B et/ou thiamine et/ou chlorhydrate de thiamine et/ou riboflavine et/ou nicotinamide et/ou vitamine C et/ou vitamine D et/ou ergocalciférol et/ou vitamine E et/ou DL-α-tocophérol et/ou acétate de tocophérol et/ou hydrogénosuccinate de tocophérol et/ou vitamine Kl et/ou vitamine P et/ou acide nicotinique et/ou pyridoxine et/ou pyridoxal et/ou pyridoxamine et/ou acide pantothénique et/ou biotine et/ou acide folique et/ou cobalamine et/ou autres substances similaires.

13. Formulation pharmaceutique ou cosmétique ou dispositif médical selon l'une des revendications 3 à 12, dans au moins une des formes sélectionnées parmi les suivantes : solution, suspension, émulsion, émulsion PIT, pâte, onguent, gel, crème, lotion, poudre, vaporisateur, savon, produit d'hygiène contenant des surfactants, huile, aérosol, bâton, shampooing et/ou produit pour la douche, bâtons de rouge à lèvre, bâtons de soins pour les lèvres, mascara, crayons pour les yeux, ombres à paupières, fards à joue, poudres, émulsions ou cires de maquillage, produits de protection de la peau, tels que les écrans solaires, avant et/ou après exposition au soleil, produits, produits pour la protection contre les agents atmosphériques, formulations adaptées à la coiffure et aux soins des cheveux tels que les shampooings, lotions, gel, spray et/ou émulsions de rinçage, produits appliqués avant ou après le shampooing, avant ou après la coloration ou la décoloration et/ou avant ou après la permanente.

14. Formulation pharmaceutique ou cosmétique ou dispositif médical selon l'une des revendications 3 à 13, comprenant au moins un véhiculeur sélectionné parmi les suivants : gras animal et/ou végétal, cires, paraffines, amidon, tragacanthe, dérivés de cellulose, glycols, silicones, bentonite, silice, talc, oxyde de zinc et/ou mélanges de ces substances, lactose, talc, silice, aluminium hydroxyde, silicate de calcium, poudre de polyamide et/ou mélanges de ces substances, propulseurs tels que les hydrofluorocarbures, hydrocarbures, éther diméthylique et/ou gaz comprimés, solvants, émulsifiants, solubilisants, glycols, huiles végétales, minérales et/ou synthétiques, glycérol, esters d'acides gras, cires et/ou mélanges de ces substances, diluants liquides tels que l'eau, l'éthanol et/ou le propylèneglycol, agents de suspension, tels que alcools éthoxylés, esters de polyoxyéthylène sorbitol, esters de polyoxyéthylène sorbitane, cellulose microcristalline, métahydroxyde d'aluminum, bentonite, agar, tragacanthe et/ou mélanges de ces substances, sels des métaux alcalins d'acides gras, sels de mono-esters d'acides gras, isothionates, lanoline, alcools gras, huiles végétales, extraits végétaux, glycérine, sucres et/ou mélanges de ces substances, sels de alcools gras sulfatés, éthers d'alcools gras sulfatés, monoester sulfosuccinates, isothionates, dérivés d'imidazoline, taurates de méthyle, sarcosinates, sulfates d'éther d'amides d'acides gras, alkylaminobétaïnes, alcools gras, glycérides d'acides gras, diéthanolamines d'acides gras, huiles végétales et synthétiques, dérivés de lanoline, esters d'acides gras avec éthoxylate de glycérol et/ou mélanges de ces substances, huiles synthétiques, esters d'acides gras, alcools gras, huiles de silicone, huiles naturelles, huiles minérales, huiles de lanolines et/ou mélanges desdites substances.
